# FASCICULE DE BREVET EUROPEEN

(11) **EP 2 051 796 B1**
(45) Date de publication et mention de la délivrance du brevet: **26.05.2010**
(21) Numéro de dépôt: 07823265.9
(22) Date de dépôt: 06.07.2007
(51) Int. Cl.: B01D 53/22, B01D 71/64

(54) **PROCEDE DE SEPARATION PAR MEMBRANE D'UNE OLEFINE CONTENUE DANS UN MELANGE D'HYDROCARBURES**
VERFAHREN ZUR MEMBRANABSCHEIDUNG EINES IN EINER MISCHUNG VON KOHLENWASSERSTOFFEN ENTHALTENEN OLEFINS
METHOD OF MEMBRANE SEPARATION OF AN OLEFIN CONTAINED IN A MIXTURE OF HYDROCARBONS

(30) Priorité: 09.08.2006 FR 0607278
(43) Date de publication de la demande: 29.04.2009
(73) Titulaire: IFP, 92852 Rueil-Malmaison Cédex (FR)
(72) Inventeur: GONZALEZ, Serge, 69150 Décines (FR); VALLET, Jacques, 69008 Lyon (FR); RODESCHINI, Hélène, 69003 Lyon (FR); BAUDOT, Arnaud, F-69390 Vernaison (FR); REYT, Jean Pierre, F-38200 Villette de Vienne (FR)
(86) Numéro de dépôt international: PCT/FR2007/001172
(87) Numéro de publication internationale: WO 2008/017743

(56) Documents cités:
- EP-A- 0 412 881
- DD-A3- 150 316
- JP-A- 1 194 905
- US-A- 4 968 331
- TANAKA, K. ET AL.: "Permeation and separation properties of polyimide membranes to olefines and paraffines" JOURNAL OF MEMBRANE SCIENCE, vol. 121, 1996, XP002434771 cité dans la demande
- SEMENOVA, S.I.: "Polymer membranes for hydrocarbon separation and removal" JOURNAL OF MEMBRANE SCIENCE, vol. 231, 2004, pages 189-207, XP002434770 cité dans la demande
- PIROUX, F. ET AL.: "Gas transport mechanism in sulfonated polyimides - Consequences on gas selectivity" JOURNAL OF MEMBRANE SCIENCE, vol. 209, 2002, pages 241-253, XP002434772

## Description

### DOMAINE DE L'INVENTION

La présente invention appartient au domaine des procédés de séparation par membrane et s'applique à la purification d'oléfines en C2 ou C3.

Le présent procédé permet plus particulièrement de séparer le propylène d'un mélange contenant d'autres hydrocarbures en C3 tel que le propane.

Les membranes utilisées dans le procédé selon la présente invention sont des membranes à base de polymère vitreux, amorphe ou pouvant présenter une très faible cristallinité, et dont le motif contient un groupement bis-phényl-9,9-fluorène.

Les membranes utilisées dans le procédé selon la présente invention présentent des valeurs de perméabilité/sélectivité supérieures à toutes les valeurs publiées dans l'art antérieur. De plus, les membranes polymères utilisées dans la présente invention conservent de très bonnes propriétés de séparation dans des conditions opératoires proches de celles employées dans l'industrie, notamment en ce qui concerne les fortes valeurs de pression partielle d'hydrocarbures, connues pour être favorables à une détérioration des performances de la plupart des membranes polymères.

Dans une version préférée de l'invention, le polymère constitutif de la couche sélective de la membrane est de type polyimide.

Plus particulièrement, le procédé selon la présente invention pourra être appliqué à la séparation d'oléfines en C2 ou C3 contenues dans un mélange de composés hydrocarbures appartenant à d'autres familles chimiques comme les paraffines.

### EXAMEN DE L'ART ANTERIEUR

Dans les documents de l'art antérieur, les performances de séparation des membranes sont généralement décrites au moyen de deux paramètres: la perméabilité et la sélectivité.

La perméabilité est définie comme la densité de flux de matière traversant la membrane, rapportée à l'épaisseur de ladite membrane, et à la différence de pression partielle des composés traversant la membrane appliquée entre les faces amont et aval.

La sélectivité de la membrane pour le constituant A par rapport au constituant B est définie comme le rapport des perméabilités des deux constituants A sur B.

La perméabilité est mesurée en barrer (1 barrer = 10⁻¹⁰ cm³.cm/cm².cm_{Hg}.s, soit en unité SI 0,75 10⁻¹⁵ Nm³.m/m².s.Pa).

Dans le cas de la séparation d'un mélange binaire, le facteur de séparation peut être calculé de deux manières; soit à partir des perméabilités obtenues en corps pur (on parle alors de sélectivité idéale ou de permsélectivité), soit à partir des données des flux en mélange (on parle alors de sélectivité en mélange ou de facteur de séparation).

Le procédé de séparation décrit dans la présente invention est réalisé par un mécanisme de solution/diffusion au travers d'un film polymère dense formant la couche sélective de la membrane.

De manière générale, les membranes offrant une grande sélectivité sont très peu perméables, et inversement, une membrane très perméable présente généralement des valeurs de sélectivité assez faibles.

De nombreux matériaux polymères mis en oeuvre sous forme de membrane ont été étudiés dans la littérature, notamment pour la séparation des oléfines contenues dans un mélange oléfines/paraffines.

Les polyimides aromatiques ont été décrits pour une utilisation dans la séparation de différents gaz. Certaines membranes polyimides aromatiques ont alors été développées dans le but de fournir des sélectivités relativement élevées, mais les perméabilités restent encore trop faibles pour une application industrielle. De nombreuses études dans ce domaine ont porté sur l'utilisation de membranes polyimides à base d'anhydride diphtalique 4,4'-(hexafluoroisopropylidène) (6FDA) car ce composé confère au matériau polymère de très bonnes propriétés filmogènes.

L'art antérieur dans le domaine des séparations par membrane appliquées à des mélanges d'hydrocarbures est assez vaste et on se limitera à l'art antérieur le plus proche représenté par le brevet US 5,749,943 qui décrit l'utilisation d'une membrane polyimide homogène ou asymétrique à base de dianhydride 6FDA et de diamine 2,2-bis(4-aminophényl)hexafluoropropane (FpDA) pour la séparation d'hydrocarbures insaturés à partir d'un mélange contenant les dits composés insaturés et des composés saturés. Des valeurs élevées de sélectivité ont été obtenues, supérieures à 30, mais les perméabilités restent faibles, inférieures à 1 barrer (1 barrer = 10⁻¹⁰ cm³.cm/cm².cm_{Hg}.s), les mesures étant effectuées à 25°C.

Tanaka *et al.* (K. Tanaka, A. Taguchi, J. Hao, H. Kita, K. Okamoto, Journal of Membrane Science 121 (1996) 197-207 et K. Okamoto, K. Noborio, J. Hao, K. Tanaka, H. Kita, Journal of Membrane Science 134 (1997) 171-179) ont étudié des membranes polyimides préparées à base de dianhydride 6FDA et de diamine 2,4,6-triméthyl-1,3-phénylènediamine (TrMPD). Ces deux publications sont extraites d'une revue dont le titre peut être traduit en français par "Journal de la Science des Membranes". les autres articles cités sont également extrait de cette même revue.

Les performances de séparation de ces membranes sont relativement bonnes. Leur coefficient de perméabilité au propylène est de 30 barrer, et le facteur de séparation idéal entre propylène et propane est de 11 à 323 K et 2 bar (1 bar = 10⁵ pascal).

Les coefficients de perméabilité des oléfines et paraffines et de sélectivité dans les polymères sont essentiellement dépendants de la pression partielle des gaz pénétrants. Par exemple, Semenova (S. I. Semenova, Journal of Membrane Science, 231 (2004) 189-207) a montré la dépendance de la perméabilité du polyimide 6FDA-TrMPD à la pression partielle en propane et propylène.

Or, les conditions de fonctionnement industrielles à forte pression sont bien connues pour être favorables au phénomène de plastification de la membrane et peuvent conduire à un déclin significatif des performances de celle-ci.

De plus, il est important de noter que la majorité des données de sélectivité publiées ont été obtenues à partir de mesures effectuées en corps pur et non pour un mélange d'hydrocarbures saturés et insaturés. D'après Tanaka *et al.* (K. Tanaka, A. Taguchi, J. Hao, H. Kita, K. Okamoto, Journal of Membrane Science 121 (1996) 197-207), la perméabilité en propylène et la sélectivité propylène/propane sont respectivement de 27 barrer et 10, lorsque les mesures sont effectuées en corps pur, à 50 °C et sous 0,2 MPa. Lorsqu'il s'agit d'un mélange de ces deux hydrocarbures, les valeurs tombent respectivement à 20 barrer et 6 en sélectivité, dans les mêmes conditions de température et de pression.

C'est pourquoi des valeurs de sélectivité obtenues en corps purs avec des composés potentiellement plastifiants ne peuvent être directement extrapolées aux mélanges, car dans les conditions de mélange, les performances de séparation des membranes polymères denses sont le plus souvent dégradées.

De manière surprenante, les propriétés de séparation des membranes polymères décrites dans la présente invention qui n'étaient pas prédictibles pour les mélanges propane/propylène, s'avèrent étonnamment bonnes. De plus, les membranes utilisées dans la présente invention permettent de travailler sous forte pression partielle d'hydrocarbures sans que leurs performances ne soient altérées.

### DESCRIPTION SOMMAIRE DE L'INVENTION

La présente invention appartient au domaine des procédés de séparation par membrane et s'applique à la séparation d'une oléfine en C2 ou C3 contenue dans un mélange d'autres composés hydrocarbures de nombre d'atomes de carbone voisin de celui de l'oléfine à séparer. Par nombre d'atomes de carbone voisin, on entend un nombre d'atome de carbone identique ou différent d'une unité de celui de l'oléfine à séparer.

Par exemple, le présent procédé permet de séparer le propylène d'un mélange contenant d'autres hydrocarbures en C3 tels que le propane.

Le procédé repose sur la perméation sélective de l'oléfine à séparer au travers d'un film dense polymère. La présence d'un groupement particulier de type bis-phényl-9,9-fluorène dans un polymère rigide mis sous forme de film dense constituant la membrane, conduit à d'excellentes propriétés de séparation, notamment en terme de perméabilité dudit film vis-à-vis de l'oléfine, tout en maintenant la sélectivité oléfine/paraffine à une valeur élevée.

Les membranes utilisées dans le procédé selon la présente invention sont des membranes de type polymère vitreux, amorphe ou présentant une faible cristallinité, comprenant, dans le motif de répétition, au moins un groupement bis-phényl-9,9-fluorène.

Le film constituant la couche sélective de la membrane polymère pourra subir un post-traitement thermique à une température supérieure à 250°C pendant au moins une heure, destiné à augmenter la sélectivité du film polymère.

L'invention consiste donc en un procédé de séparation par membrane dans lequel la couche sélective de la membrane polymère est constituée d'un film dense polymère dont la structure chimique contient un groupement bis-phényl-9,9-fluorène.

La couche sélective de la membrane polymère contenant le groupement bis-phényl-9,9-fluorène est sélectionnée dans le groupe constitué par les polymères des familles suivantes: les polyimides, les polyamides, les polycarbonates, les polysulfones, les poly(amides imides), les poly(éther sulfones), les polyesters, ou par les copolymères ou mélanges de polymères de ces familles.

De façon préférée, la couche sélective de la membrane polymère contenant le groupement bis-phényl-9,9-fluorène appartient à la famille des polyamides.

De façon également préférée, la couche sélective de la membrane polymère contenant le groupement bis-phényl-9,9-fluorène appartient à la famille des polycarbonates.

De façon encore davantage préférée, la couche sélective de la membrane polymère contenant le groupement bis-phényl-9,9-fluorène appartient à la famille des polyimides.

### DESCRIPTION DETAILLEE DE L'INVENTION

La présente invention appartient au domaine des procédés de séparation par membrane et s'applique à la séparation d'une oléfine en C2 ou C3 (c'est-à-dire une oléfine comprenant 2 ou 3 atomes de carbone) contenue dans un mélange d'autres composés hydrocarbures de nombre d'atomes de carbone voisin de celui de l'oléfine à séparer.

Par exemple, le présent procédé permet de séparer le propylène d'un mélange contenant d'autres hydrocarbures en C3 tels que le propane.

Le procédé repose sur la perméation sélective de l'oléfine à séparer au travers d'un film dense polymère. La présence d'un groupement particulier de type bis-phényl-9,9-fluorène dans un polymère rigide mis sous forme de film dense constituant la membrane, conduit à d'excellentes propriétés de séparation, notamment en terme de perméabilité dudit film vis-à-vis de l'oléfine, et ce tout en maintenant une sélectivité oléfine/paraffine élevée.

Les membranes utilisées dans le procédé selon la présente invention sont des membranes de type polymère amorphe, vitreux ou présentant une faible cristallinité, comprenant, dans le motif de répétition, au moins un groupement bis-phényl-9,9-fluorène.

L'invention consiste donc en un procédé de séparation par membrane dans lequel la couche sélective de la membrane polymère est constituée d'un film dense polymère dont la structure chimique contient un groupement bis-phényl-9,9-fluorène.

La couche sélective de la membrane polymère contenant le groupement bis-phényl-9,9-fluorène est sélectionnée dans le groupe constitué par les polymères des familles suivantes: les polyimides, les polyamides, les polycarbonates, les polysulfones, les poly(amides imides), les poly(éther sulfones), les polyesters, ou par les copolymères ou mélanges de polymères de ces familles.
- Dans une première variante de l'invention, la couche sélective de la membrane polymère contenant le groupement bis-phényl-9,9-fluorène appartient à la famille des polyimides.
- Dans une seconde variante de l'invention, la couche sélective de la membrane polymère contenant le groupement bis-phényl-9,9-fluorène appartient à la famille des polyamides.
- Dans une troisième variante de l'invention, la couche sélective de la membrane polymère contenant le groupement bis-phényl-9,9-fluorène appartient à la famille des polycarbonates.
- Dans une quatrième variante de l'invention, la couche sélective de la membrane polymère contenant le groupement bis-phényl-9,9-fluorène appartient à la famille des polysulfones.
- Dans une cinquième variante de l'invention, la couche sélective de la membrane polymère contenant le groupement bis-phényl-9,9-fluorène appartient à la famille des poly(amides imides).
- Dans une sixième variante de l'invention, la couche sélective de la membrane polymère contenant le groupement bis-phényl-9,9-fluorène appartient à la famille des poly(éther sulfones).
- Dans une septième variante de l'invention, la couche sélective de la membrane polymère contenant le groupement bis-phényl-9,9-fluorène appartient à la famille des polyesters.

De façon très préférée, la couche sélective de la membrane polymère contenant le groupement bis-phényl-9,9-fluorène appartient à la famille des polyimides.

Le polymère constitutif de la couche sélective de la membrane peut être un homopolymère, un copolymère, ou un mélange de polymères.

Les membranes utilisées dans la présente invention, outre la présence du polymère vitreux comprenant dans le motif de répétition au moins un groupement bis-phényl-9,9-fluorène, pourront contenir des charges minérales et organiques et des additifs destinés à entraîner une amélioration du facteur de séparation et/ou à favoriser la perméabilité.

On peut à titre d'exemple, citer les charges minérales telles que les sels métalliques, les zéolites, les argiles, les composés mésoporeux, les silices natives ou post-traitées, les noirs de carbone, les polymères pyrolysés, les nanotubes de carbones, les dendrimères.

Les membranes utilisées dans la présente invention, en plus du polymère vitreux comprenant dans le motif de répétition au moins un groupement bis-phényl-9,9-fluorène, pourront également contenir des agents de réticulation permettant une amélioration du facteur de séparation et/ou de la perméabilité.

Les membranes utilisées dans la présente invention pourront également être traitées chimiquement, thermiquement, ou par rayonnement, permettant ainsi d'améliorer le facteur de séparation et/ou de favoriser la perméabilité.

Le polymère constitutif de la couche sélective de la membrane selon l'invention comporte dans le motif de répétition au moins un groupement bis-phényl-9,9-fluorène de formule chimique générale : 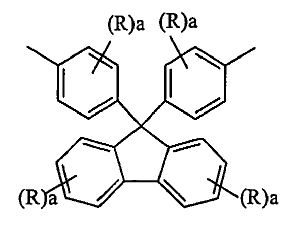 dans lequel, chacun des groupes R représente soit un groupe alkyle, linéaire ou ramifié ayant de 1 à 16 atomes de carbone, soit un groupe alkoxy, linéaire ou ramifié ayant de 1 à 16 atomes de carbone. L'indice a peut prendre pour chacun des groupes R et de manière indépendante d'un groupe à l'autre, soit la valeur zéro, soit une valeur entière comprise entre 1 et 4. Préférentiellement chaque valeur de l'indice a sera 0 ou 1.

De manière encore plus préférée, l'indice a aura une valeur nulle, ce qui revient à la suppression des groupes R.

Pour les groupes alkyle, on peut citer de manière non limitative les groupes méthyle, éthyle, propyle, isopropyle, les groupes butyle linéaires ou ramifiés.

Pour les groupes alkoxy, on peut citer de manière non limitative les groupes méthoxy, éthoxy, propyloxy, et les groupes butyloxy linéaires ou ramifiés.

Dans une version préférée, le polymère constitutif de la couche sélective de la membrane sera un homopolymère, ou un copolymère de formule générale :

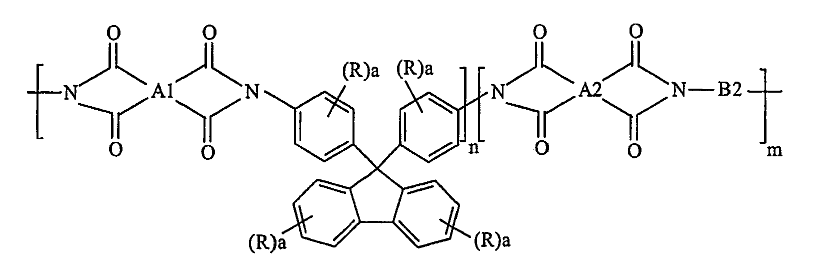

Où A1 et A2 sont des groupements organiques tétravalents hydrocarbonés choisis parmi des groupes d'hydrocarbures aromatiques, alicycliques et aliphatiques, et le groupe B2 est un groupement organique bivalent hydrocarboné choisi parmi des groupes d'hydrocarbures aromatiques, alicycliques et aliphatiques. Les indices m et n représentent un nombre entier positif qui correspond au degré de polymérisation.

Dans une version préférée, le polyimide constitutif de la couche sélective de la membrane est un polymère statistique, alterné, séquencé ou block.

La voie la plus généralement utilisée pour l'obtention du polyimide constitutif de la couche sélective de la membrane résulte de la réaction chimique entre:
- une diamine comportant dans sa structure le groupement bis-phényl-9,9-fluorène de formule générale :

   H₂N-B2-NH₂
- et un dianhydride de formule générale :

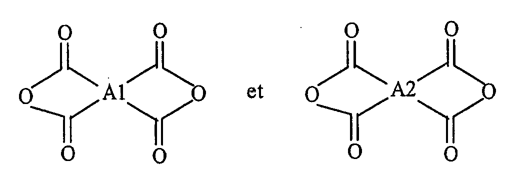

On pourra dans le cadre de l'invention utiliser un précurseur tel que par exemple un tétra acide carboxylique, ou l'hémiester d'un tétra acide carboxylique.

Dans le cadre de l'invention la diamine peut être choisie dans la liste suivante:
- la 1,4-diamino-2,3,5,6-tétraméthylbenzène
- bis(4-aminophényl) éther
- 2,4-diamino-1-isopropylbenzène
- les diaminoanthraquinones
- 2,7-diaminofluorène
- 4,4'-diamino-3,3'-diméthoxybiphényl
- 2,4-diaminotoluène
- diaminodiphénylsulfone
- bis[4-(4-aminophénoxy)phényl]sulfone
- 9,10-bis(4-aminophényl)anthracène
- 1,4-bis(4-aminophényl)benzène
- bis(4- aminophényl)méthane
- bis(4-amino-3-éthylphényl)méthane
- bis(4-amino-3-méthylphényl)méthane
- bis(4-amino-3-chloro-phényl)méthane
- bis(4-aminophényl)sulfide
- 2,2-bis(4-amino-3-hydroxyphényl)propane
- 4,4'-diamino-3,3'-dichlorobiphényl
- 4,4'-diamino-3,3'-dihydroxybiphényl
- 4,4'-diaminobiphényl
- 9,9-bis(4-aminophényl)fluorène
- bis(4-amino-2,6-méthylphényl) méthane
- 1,4-diamino-2,5-dichlorobenzène
- 1,4-diamino-2,5-diméthylbenzène
- 1,3-diamino-2,4,6-triméthylbenzène
- bis(3-aminopropyl)tétraméthyldisiloxane
- 2,5-diaminopyridine
- 4,4'-diaminobenzanilide
- 1,5-diaminonaphthalène
- 1,3- diamino-5-trifluorométhylbenzène
- 4,4'-diamino-3,3',5,5'-tétraméthylbiphényl
- 3,3'-diamino-4,4'-dihydroxybiphényl
- 1,3-phénylènediamine
- 1,4-phénylènediamine
- 1,4-bis(4-aminophenoxy)benzène.
-

De manière préférée on choisira les diamines dans la liste suivante:
- la 1,4-diamino-2,3,5,6-tétraméthylbenzène
- 9,9-bis(4-aminophényl)fluorène
- 1,3-diamino-2,4,6-triméthylbenzène
- bis(3-aminopropyl)tétraméthyldisiloxane

Dans le cadre de l'invention, le dianhydride choisi peut être choisie dans la liste suivante:
- le dianhydride de l'acide bis(3,4-dicarboxyphényl)sulfone
- le dianhydride de l'acide 2,2-bis(3,4-dicarboxyphényl)hexafluoropropane
- le dianhydride de l'acide 1,1-bis(3,4-dicarboxyphényl)éthane
- l'anhydride pyromellitique
- le dianhydride de l'acide 2,3,6,7-naphthalenetétracarboxylique
- le dianhydride de l'acide 3,3',4,4'-biphényltétracarboxylique
- le dianhydride de l'acide 1,2,5,6-naphthalenetétracarboxylique
- le dianhydride de l'acide 2,2'3,3'-biphényltétracarboxylique
- le dianhydride de l'acide 3,3',4,4'-benzophenonetétracarboxylique
- le dianhydride de l'acide oxydiphthalique
- le dianhydride de l'acide 1,4,5,8-naphthalènetétracarboxylique
- le dianhydride de l'acide 2,2-bis(3,4-dicarboxyphényl)propane
- le dianhydride de l'acide 3,4,9,10-pérylènetétracarboxylique
- le dianhydride de l'acide 1,1-bis(2,3-dicarboxyphényl)éthane
- le dianhydride de l'acide bis(2,3-dicarboxyphényl)méthane
- le dianhydride de l'acide bis(3,4-dicarboxyphényl)méthane.

On choisira de manière préférée le dianhydride dans la liste suivante:
- le dianhydride de l'acide 2,2-bis(3,4-dicarboxyphényl)hexafluoropropane
- l'anhydride pyromellitique
- le dianhydride de l'acide 3,3',4,4'-biphényltétracarboxylique
- le dianhydride de l'acide 3,3',4,4'-benzophenonetétracarboxylique

Les solvants nécessaires à la réalisation de la polymérisation peuvent être choisi dans la liste suivante:
- N,N-diméthylformamide
- N,N-diéthylformamide
- N,N- diméthylacétamide (DMAC)
- N,N-diéthylacétamide
- N-méthyl-2-pyrrolidone (NMP)
- N-cyclohexyl-2-pyrrolidone
- phénol
- o-, m-, p-crésol
- xylénol
- phénols hallogénés
- catéchol
- hexaméthylphosphoramide
- diméthylpropyl urée
- alcools benzyliques
- lactates
- lactones telles que la γ- butyrolactone.

Les solvants seront choisis de manière préférée dans la liste suivante:
- N,N- diméthylacétamide (DMAC)
- N-méthyl-2-pyrrolidone (NMP)
- o-, m-, p-crésol
- lactones telles que la γ- butyrolactone.

Ces solvants peuvent être utilisés seuls ou en mélange.

La connaissance de la masse moléculaire du polymère n'est pas indispensable et l'on préférera suivre l'évolution de la viscosité inhérente du polymère qui doit être au moins supérieure à 0,1 dl/g, et préférablement comprise entre 0,3 dl/g et 2 dl/g. La viscosité inhérente se définit par rapport à une viscosité de référence et à la concentration du polymère en solution dans le solvant. Sa valeur est homogène à l'inverse de ladite concentration soit 1d1/g (=0,1 m³/kg).

La plupart des polymères envisagés pour une mise en oeuvre sous forme de membrane dans la présente invention sont solubles dans une grande variété de solvants organiques incluant la plupart des solvants aprotiques, qui sont généralement utilisés pour la formation de membranes polymères comme la NMP.

La membrane polymère qui contient le groupement bis-phényl-9,9-fluorène peut être homogène ou asymétrique.

Dans une variante du procédé selon l'invention, le polymère constitutif de la couche sélective de la membrane sera un polyimide issu de la polycondensation d'une part du dianhydride de l'acide 3,3',4,4'-biphényltétracarboxylique, et d'autre part d'un mélange de la diamine 9,9-bis(4-aminophényl)fluorène et de la diamine 1,3-diamino-2,4,6-triméthylbenzène.

Dans une autre variante du procédé selon l'invention, le polymère constitutif de la couche sélective de la membrane est un polyimide issu de la polycondensation d'une part du dianhydride d de l'acide 3,3',4,4'-benzophénonetétracarboxylique et d'autre part d'un mélange de la diamine 9,9-bis(4-aminophényl)fluorène et de la diamine 1,3-diamino-2,4,6-triméthylbenzène.

Le polymère constitutif de la couche sélective de la membrane peut être mis en oeuvre sous forme de film ou de fibres selon les techniques connues de l'homme du métier.

Une fois synthétisé, le polymère sous la forme d'un solide est dissout dans un solvant approprié comme la NMP par exemple, à une teneur en polymère de l'ordre de 1% à 50% en masse, et préférablement comprise entre 5% et 20% en masse.

La solution est étendue sous forme de film à l'épaisseur désirée sur un support plan ou sur un support se présentant sous forme de fibres creuses, ou bien est extrudée au travers d'une fileuse conventionnelle.

Il est possible de réaliser la membrane, et l'on parlera alors de membrane composite, en déposant un film de polymère comportant dans sa chaîne au moins un groupement bis-phényl-9,9-fluorène d'une épaisseur comprise entre 0,05 et 1 micron (1 µm = 10⁻⁶ mètre) sur un support préalablement mis en oeuvre sous forme de fibre creuse.

On choisira avantageusement le support de telle manière qu'il présente l'avantage d'être beaucoup plus perméable que les polyimides en général, et ne contribue pas significativement à la résistance au transfert de matière à travers la membrane composite résultante.

Selon un mode de réalisation de l'invention, le support sera une couche poreuse ou une fibre creuse constituée d'un matériau polymère tel que par exemple un polysulfone, un polyéthersulfone, un polyétherimide, un polyfluorure de vinylidène, un poléthylène ou un polypropylène, un polyacrylonitrile, un polyimide, un polyoxyde de phénylène, un polymère dérivé de la cellulose tel qu'un acétate de cellulose ou un éthylcellulose. Le support pourra être un polymère constitué de différents matériaux organiques ou minéraux.

L'adhésion entre la couche sélective et le support nécessite dans certains cas des traitements physiques ou chimiques qui sont bien connus de l'homme de l'art.

Dans la suite du texte on prendra comme exemple la séparation du propylène contenu dans un mélange de propylène et de propane. Mais il convient de garder à l'esprit que le présent procédé s'applique aussi bien à la séparation de l'éthylène d'un mélange contenant d'autres hydrocarbures en C2.

Les membranes de la présente invention peuvent être utilisées dans divers types de modules destinés à réaliser l'unité de séparation. Le module de séparation final peut être constitué d'une ou de plusieurs membranes. Le module peut être assemblé à d'autres modules identiques de manière à former une unité de séparation ayant la taille désirée.

En fonctionnement, le mélange contenant l'oléfine à séparer est mis en contact avec un des cotés de la membrane. En imposant une différence de pression entre le coté de la charge et le coté perméat, les composés oléfiniques traversent la membrane à une vitesse plus importante que les paraffines comprenant le même nombre d'atomes de carbone. Ainsi, une oléfine en C3 traverse plus rapidement la membrane qu'une paraffine en C3. Cette différence de vitesse produit un flux hydrocarboné enrichi en oléfine, qui est prélevé du coté perméat de la membrane.

La présente invention n'est pas destinée uniquement aux séparations en phase gaz mais peut être étendue à d'autres types de séparation, en phase liquide par exemple, et ceci pour des conditions de température et de pression couvrant un large domaine d'utilisation. En outre, la séparation peut avoir lieu pour des mélanges contenant plus de deux composants.

De manière large le procédé de séparation par membrane selon l'invention fonctionne à une température comprise entre -80 °C et 200 °C, et à une pression comprise entre 0,1 MPa et 10 MPa. La pression est entendue comme celle du mélange à séparer.

Le plus souvent le procédé selon l'invention fonctionne à une température comprise entre -60 °C et 100°C et à une pression comprise entre 0,1 MPa et 5 MPa.

De manière préférée le procédé selon l'invention fonctionne à une température comprise entre 30°C et 80 °C, et à une pression est comprise entre 0,1 MPa et 3 MPa. Et de manière également préférée, le procédé selon l'invention fonctionne à une température comprise entre 40°C et 70 °C, et à une pression comprise entre 1 MPa et 2 MPa. Dans les conditions opératoires du procédé selon l'invention, la pression partielle en oléfine est généralement supérieure à 0,3 MPa.

### EXEMPLES SELON L'INVENTION

### Exemple 1 (selon l'invention)

Le polymère qui fait l'objet de l'exemple 1 est le résultat de la polycondensation du dianhydride de l'acide hexafluoropropylidène-4,4'-diphtalique (6FDA) et de la 9,9-bis(4-aminophényl)fluorène (BDAF) en mélange équimolaire.

Après purification des monomères par recristallisation dans des solvants appropriés, la polycondensation du polyimide est réalisée en deux étapes: dans un premier temps, le polyamide acide est réalisé à température ambiante, puis le polyimide est obtenu dans une deuxième étape de cyclisation par voie chimique.

Lors de la première étape de polymérisation, le mélange de la dianhydride et de la diamine est effectué sous atmosphère inerte et en milieu anhydre dans le solvant N-N-diméthylacétamide (DMAC). L'étape de cyclo-déshydratation est effectuée par ajout goutte à goutte d'un mélange cyclisant composé de triéthylamine et d'anhydride acétique en mélange dans le solvant de synthèse.

Le polyimide ainsi obtenu est alors précipité dans de l'eau puis broyé. Le polymère solide est ensuite filtré, rincé, puis séché à l'étuve sous vide en augmentant progressivement la température jusqu'à atteindre 150 °C. La viscosité inhérente du polymère ainsi obtenu est de 1,3 dl/g.

Le matériau sous forme de broyé est ensuite mis en solution dans de la DMAC à une concentration massique de 12% sous l'effet d'une bonne agitation mécanique à température ambiante.

La solution limpide est ensuite filtrée sous une pression de 0,2 MPa sur un filtre de type Millipore ayant un seuil de coupure de 1 µm. Cette solution est ensuite mise sous forme d'un film à l'aide d'une barre spiralée de 300 µm sur une plaque de verre préalablement dégraissée i l'acétone puis séchée.

La plaque est introduite dans une étuve. L'évaporation du solvant est effectuée par une élévation progressive de la température jusqu'à 200°C. La température finale est maintenue en palier pendant deux heures. Après refroidissement, la plaque est immergée dans l'eau où l'on observe le décollement du film.

Après évaporation du solvant, le film obtenu présente une épaisseur moyenne de 20 µm.

Jn échantillon de ce film a ensuite été testé dans une cellule de perméation circulaire d'un diamètre efficace de 5,5 cm placé dans une enceinte thermostatée.

La face amont de la membrane ainsi testée est balayée pendant 72 h avec un flux gazeux de 0 Nl/h composé de propylène et de propane, alors que le compartiment en aval de la membrane, dans lequel est collecté le perméat, est balayé par un flux d'azote de 1 Nl/h à la pression atmosphérique.

La composition des différents fluides entrant et sortant des différents compartiments de la cellule de perméation est obtenue par chromatographie en phase gazeuse.

Les performances en régime permanent du film ainsi testé sont les suivantes :

**Tableau 1. Performances de la membrane polyimide 6FDA-BDAF (Pression charge = 0,9 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| 90/10 | 0,8 MPa | 50°C | 84 barrer | 7 |
| 70/30 | 0,8 MPa | 50°C | 80 barrer | 6,2 |

**Tableau 2. Performances de la membrane polyimide 6FDA-BDAF (Pression charge = 1,7 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| 90/10 | 0,8 MPa | 60°C | 90 barrer | 6,8 |
| 70/30 | 0,8 MPa | 60°C | 85 barrer | 6 |

### Exemple 2 (selon l'invention)

La synthèse du polyimide est réalisée en deux étapes. Lors de la première étape, le dianhydride de type 6FDA est mis en contact avec la BDAF dans le solvant N-méthylpyrrolidone (NMP) sous atmosphère inerte et en milieu anhydre.

Après 3 heures d'agitation à température ambiante, le polyamide acide est obtenu.

La seconde étape consiste en une déshydratation thermique de ce polyamide acide par chauffage (30 min à 100 °C, 1 heure à 160 °C, 1 heure à 180 °C et deux heures à 200 °C).

Le polyimide ainsi obtenu est alors précipité dans de l'eau, broyé, et séché comme décrit dans l'exemple 1 de la présente invention. La viscosité inhérente du polymère ainsi obtenu était de 0,55 dl/g.

Le polymère est ensuite redissout dans le solvant NMP à une concentration de 10 % massique. La solution limpide est ensuite filtrée sous une pression de 0,2 MPa sur un filtre de type Millipore ayant un seuil de coupure de 1 µm. Cette solution est ensuite mise sous forme d'un film à l'aide d'une barre spiralée de 300 µm sur une plaque de verre préalablement dégraissée à l'acétone puis séchée.

La plaque est introduite dans une étuve. L'évaporation du solvant est effectuée par une élévation progressive de la température jusqu'à 200°C.

Après évaporation du solvant, le film obtenu présente une épaisseur moyenne de 32 µm.

Les performances du film selon l'exemple 2 pour la séparation propylène/propane ont été obtenues dans des conditions de tests identiques à celles décrites dans l'exemple 1.

**Tableau 3. Performances de la membrane polyimide 6FDA-BDAF (Pression charge = 0,9 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| mélange 90/10 | 0,8 MPa | 50°C | 3,0 barrer | 12 |
| mélange 70/30 | 0,8 MPa | 50°C | 3,4 barrer | 10 |

**Tableau 4. Performances de la membrane polyimide 6FDA-BDAF (Pression charge = 1,7 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| 90/10 | 0,8 MPa | 60°C | 3,2 barrer | 10 |
| 70/30 | 0,8 MPa | 60°C | 3,0 barrer | 8 |

### Exemple 3 (exemple selon l'invention)

La synthèse du film polyimide est réalisée selon le procédé décrit dans l'exemple 2. Après évaporation du solvant, le film obtenu présente une épaisseur moyenne de 30 µm.

Le film une fois séché est recuit en étude sous azote à 350 °C pendant 2 heures.

Les performances du film obtenu selon l'exemple 3 pour la séparation propylène/propane ont été mesurées dans des conditions de tests identiques à celles décrites dans l'exemple 1.

**Tableau 5. Performances de la membrane polyimide 6FDA-BDAF selon l'exemple 3 (Pression charge = 0,9 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| mélange 90/10 | 0,8 MPa | 50°C | 1,8 barrer | 13 |
| mélange 70/30 | 0,8 MPa | 50°C | 1,3 barrer | 12 |

Si l'on compare les performances de la membrane décrite dans l'exemple 2 (sans post-traitement thermique) et de la membrane décrite dans l'exemple 3 synthétisée de façon analogue mais ayant subi un post-traitement thermique, il est clair que la recuisson du film membranaire conduit à une augmentation de la sélectivité en mélange.

### Exemple 4 (exemple selon l'invention)

Le film polymère BTDA-BDAF faisant l'objet de l'exemple 4 est le résultat de la polycondensation du dianhydride de l'acide 3,3',4,4'-benzophénonetétracarboxylique (BTDA) et de la 9,9-bis(4-aminophényl)fluorène (BDAF) en mélange équimolaire.

Après purification des monomères par recristallisation dans des solvants appropriés, la polycondensation du polyimide BTDA-BDAF est réalisée en deux étapes: dans un premier temps, le polyamide acide est réalisé, puis le polyimide est obtenu dans une deuxième étape de cyclisation obtenue par voie chimique.

Lors de la première étape de polymérisation, le mélange de la dianhydride et de la diamine est effectué sous atmosphère inerte et en milieu anhydre dans le solvant NMP.

L'étape de cyclo-déshydratation est effectuée par ajout goutte à goutte d'un mélange cyclisant composé de triéthylamine et d'anhydride acétique en mélange dans le solvant de synthèse.

Le polyimide ainsi obtenu est alors précipité dans de l'eau puis broyé. Il est ensuite filtré, rincé, puis séché à l'étuve sous vide en augmentant progressivement la température jusqu'à atteindre 150 °C. La viscosité inhérente du polymère ainsi obtenu est de 0,8 dl/g dans la NMP.

Le matériau sous forme broyé est ensuite mis en solution dans de la NMP à une concentration massique de 10 % sous l'effet d'une bonne agitation mécanique à température ambiante.

La solution limpide est ensuite filtrée sous une pression de 0,2 MPa sur un filtre de type Millipore ayant un seuil de coupure de 1 µm. Cette solution est ensuite mise sous forme d'un film à l'aide d'une barre spiralée de 300 µm sur une plaque de verre préalablement dégraissée à l'acétone puis séchée.

La plaque est introduite dans une étuve. L'évaporation du solvant est effectuée par une élévation progressive de la température jusqu'à 200°C. La température finale est maintenue en palier pendant deux heures. Après refroidissement, la plaque est immergée dans l'eau où l'on observe le décollement du film.

Après évaporation du solvant, le film obtenu présente une épaisseur moyenne de 24 µm.

Les performances du film obtenu selon l'exemple 4 pour la séparation propylène/propane ont été obtenues dans des conditions de tests identiques à celles décrites dans l'exemple 1.

**Tableau 6. Performances de la membrane polyimide BTDA-BDAF (Pression charge = 0,9 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| mélange 90/10 | 0,8 MPa | 50°C | 4 barrer | 12 |
| mélange 70/30 | 0,8 MPa | 50°C | 3 barrer | 12 |

### Exemple 5 (selon l'invention)

Le film polymère BPDA-BDAF faisant l'objet de l'exemple 5 est le résultat de la polycondensation du dianhydride de l'acide 3,3',4,4'-bis-phényltétracarboxylique (BPDA) et de la 9,9-bis(4-aminophényl)fluorène (BDAF) en mélange équimolaire.

Les monomères sont introduits dans le solvant DMAC sous atmosphère inerte et en milieu anhydre.

Après de 8 heures d'agitation à température ambiante, le polyamide acide est obtenu.

La seconde étape consiste en une déshydratation thermique de ce polyamide acide sous forme de film par chauffage (une heure à 100 °C, trois heures à 200 °C) dans une étuve .

Après évaporation du solvant, le film obtenu présente une épaisseur moyenne de 40 µm.

Les performances du film obtenu selon l'exemple 5 pour la séparation propylène/propane ont été obtenues dans des conditions de tests identiques à celles décrites dans l'exemple 1.

**Tableau 7. Performances de la membrane polyimide BPDA-BDAF (Pression charge = 0,9 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| mélange 90/10 | 0,8 MPa | 50°C | 2 barrer | 20 |
| mélange 70/30 | 0,8 MPa | 50°C | 1,5 barrer | 16 |

### Exemple 6 (selon l'invention)

Le polymère 6FDA-BDAF/TrMPD (50/50) faisant l'objet de l'exemple 6 est le résultat de la polycondensation en mélange équimolaire du dianhydride de l'acide hexafluoropropylidène-4,4'-diphtalique (6FDA) et deux diamines en proportion molaire 50/50, la 9,9-bis(4-uninophényl)fluorène (BDAF) et la 2,4,6-triméthylphénylènediamine (TrMPD).

Après purification des monomères par recristallisation dans des solvants appropriés, la polycondensation du polyimide est réalisée en deux étapes: dans un premier temps, le polyamide acide est réalisé à température ambiante, puis le polyimide est obtenu dans une deuxième étape de cyclisation par voie chimique.

Lors de la première étape de polymérisation, le mélange de la dianhydride et des diamines est effectué sous atmosphère inerte et en milieu anhydre dans le solvant DMAC.

L'étape de cyclo-déshydratation est effectuée par ajout goutte à goutte d'un mélange cyclisant composé de triéthylamine et d'anhydride acétique en mélange dans le solvant de synthèse.

Le polyimide ainsi obtenu est alors précipité dans de l'eau puis broyé. Le polymère solide est ensuite filtré, rincé, puis séché à l'étuve sous vide en augmentant progressivement la température jusqu'à atteindre 150 °C. La viscosité inhérente du polymère ainsi obtenu est de 0,5 dl/g.

Le matériau sous forme de broyé est ensuite mis en solution dans de la DMAC à une concentration massique de 12 % sous l'effet d'une bonne agitation mécanique à température ambiante.

La solution limpide est ensuite filtrée sous une pression de 0,2 MPa sur un filtre de type Millipore ayant un seuil de coupure de 1 µm. Cette solution est ensuite mise sous forme d'un film à l'aide d'une barre spiralée de 300 µm sur une plaque de verre préalablement dégraissée à l'acétone puis séchée.

La plaque est introduite dans une étuve. L'évaporation du solvant est effectuée par une élévation progressive de la température jusqu'à 200°C. La température finale est maintenue en palier pendant deux heures. Après refroidissement, la plaque est immergée dans l'eau où l'on observe le décollement du film. Après évaporation du solvant, le film obtenu présente une épaisseur moyenne de 23 µm.

Les performances en régime permanent du film obtenu selon l'exemple 6 pour la séparation propylène/propane ont été mesurées dans des conditions de tests identiques à celles décrites dans l'exemple 1.

**Tableau 8. Performances de la membrane polyimide 6FDA-BDAF/TrMPD (50/50) ( Pression charge = 0,9 MPa)**

| Composition de la charge (% propylène; % propane) | Différence de pression entre les faces amont et aval du film | Température du film | Perméabilité C₃H₆ | Perméabilité C₃H₆ / Perméabilité C₃H₈ (sélectivité en mélange) |
|---|---|---|---|---|
| 90/10 | 0,8 MPa | 60°C | 22 barrer | 7 |
| 70/30 | 0,8 MPa | 60°C | 20 barrer | 7 |

### Exemple n°7 (selon l'art antérieur)

Le polyimide de type 6FDA-TrMPD a été identifié dans l'art antérieur comme offrant des propriétés supérieures à tous les autres polyimides évalués dans l'art antérieur pour la séparation propylène/propane, car il présente une perméabilité au propylène supérieure à 1 barrer. De plus, ce polymère offre une sélectivité propylène/propane supérieure à 10 (sélectivité idéale). Ces performances sont résumées par les tableaux 9 et 10 ci dessous:

**Tableau 9. Perméabilités du polyimide de type 6FDA-TrMPD au propylène et au propane mesurées lors d'expériences de perméation en corps pur à 50°C (Tanaka, K.; Taguchi, A.; Hao, J.; Kita, H.; Okamoto, K., Journal of Membrane Science, 121 (1996) 197-207).**

| Pression (bar) | Perméabilité propylène (barrer) | Perméabilité propane (barrer) | Perméabilité C₃H₆ / Perméabilité C₃H₈ (sélectivité idéale) |
|---|---|---|---|
| 1 | 30 | 2,1 | 14,3 |
| 1,5 | 27 | 2 | 13,5 |
| 2 | 26 | 2 | 13,0 |
| 3 | 24 | 1,8 | 13,3 |
| 5 | 22 | 1,9 | 11,6 |
| 6 | 23 | 2 | 11,5 |
| 7 | 25** | 2,2 | 11,4 |

| | | | |
|---|---|---|---|
| ** valeur extrapolée | | | |

La dégradation des performances du polyimide de type 6FDA-TrMPD aux pressions croissantes du fait des mécanismes de plastification est encore plus marquée lors des tests de séparation propylène/propane en mélange menés par Tanaka et al. 1996. Le tableau 10 montre en effet que la sélectivité en mélange du 6FDA-TrMPD est presque deux fois plus faible que la sélectivité idéale vis-à-vis du mélange propylène/propane.

**Tableau 10. Comparaison des performances idéales et en mélange du polyimide de type 6FDA-TrMPD pour la séparation propylène/propane (Tanaka, K.; Taguchi, A.; Hao, J.; Kita,H.; Okamoto, K., Journal of Membrane Science, 121 (1996) 197-207).**

| Composition de la charge (% propylène; % propane) | Différence de pression entre les faces amont et aval du film | Température du film | Perméabilité C₃H₆ (barrer) | Perméabilité C₃H₆ / Perméabilité C₃H₈ |
|---|---|---|---|---|
| composés purs | 0,4 MPa | 50°C | 27 barrer | 10* |
| mélange 50/50 | 0,4 MPa | 50°C | 20 barrer | 6** |
| composés purs | 0,6 MPa | 50°C | 25 barrer | 9* |
| mélange 50/50 | 0,6 MPa | 50°C | 20 barrer | 5** |

| | | | | |
|---|---|---|---|---|
| *: sélectivité idéale **: sélectivité en mélange | | | | |

Comme le montrent clairement les tableaux 9 et 10 ci dessus, les performances du polymère 6FDA-TrMPD sont plus faibles que celles des polymères présentés dans les exemples 1 à 6 selon la présente invention.

Les matériaux revendiqués dans la présente invention sont en effet :
· soit plus sélectifs que le meilleur polyimide de l'art antérieur décrit dans le tableau 9 (exemples 2 à 5) tout en offrant une perméabilité vis-à-vis du propylène supérieure à 1 barrer.
· soit plus perméable (d'un facteur 4) que le meilleur polyimide de l'art antérieur décrit dans le tableau 9 (exemple 1), tout en offrant une sélectivité propylène/propane en mélange comparable dans des conditions opératoires favorisant davantage les mécanismes de plastification.

### Exemple 8 (comparatif):

Afin de confirmer le gain offert par le polyimide de type 6FDA-BDAF par rapport au polyimide de type 6FDA-TrMPD pour la séparation propylène/propane, il a été choisi de synthétiser un film de 6FDA-TrMPD afin d'évaluer ses performances dans des conditions opératoires similaires à celles décrites dans les exemples selon l'invention 1 à 6.

La synthèse du polymère de type 6FDA-TrMPD a été réalisée de la façon suivante :
Après 2 heures à température ambiante, le mélange de NMP, diamine TrMPD et dianhydride est porté à 100°C.

Après 1 heure à 100°C, le mélange est porté par pallier à 200°C. A cette température, on observe une augmentation progressive de la viscosité du mélange. Le polymère est précipité dans l'eau, lavé puis séché pendant 6 heures à 80°C sous vide. La viscosité inhérente du polymère dans la NMP ainsi obtenu est de 0,54 dl/g.

Le matériau est mis en solution dans la NMP à une concentration massique de 10 % sous l'effet d'une bonne agitation mécanique à température ambiante. La solution limpide est ensuite filtrée sous une pression de 0,2 MPa sur un filtre de type millipore ayant un seuil de coupure de 1 µm.

Cette solution est ensuite filmée à l'aide d'une barre spiralée de 300 µm sur une plaque de verre préalablement dégraissée à l'acétone puis séchée.

La plaque est introduite dans une étuve. L'évaporation du solvant est effectuée par une élévation progressive de la température jusqu'à 200°C.

La température finale est maintenue en palier pendant une heure. Après refroidissement, la plaque est immergée dans l'eau ou l'on observe le décollement du film. Le film est ensuite séché en étuve à 50°C pendant 24 heures.

Après évaporation du solvant, le film obtenu présentait une épaisseur moyenne de 35 µm.

Les performances du film obtenu selon l'exemple 8 pour la séparation propylène/propane ont été obtenues dans des conditions de tests identiques à celles décrites dans l'exemple n°1.

Les performances obtenues avec le film à base de 6FDA-TrMPD fabriqué dans les conditions décrites ci-dessus sont proches de celles des films à base de 6FDA-TrMPD décrites par Tanaka et al. 1996.

**Tableau 11. Performances du polyimide 6FDA-TrMPD (Pression charge = 0,9 MPa)**

| Composition de la charge (% propylène; % propane) | Différence de pression entre les faces amont et aval du film | Température du film | Perméabilité C₃H₆ (barrer) | Perméabilité C₃H₆ / Perméabilité C₃H₈ (sélectivité en mélange) |
|---|---|---|---|---|
| 90/10 | 0,8 MPa | 50°C | 15 barrer | 6 |
| 70/30 | 0,8 MPa | 50°C | 16 barrer | 6 |

**Tableau 12. Performances du polyimide 6FDA-TrMPD (Pression charge = 1,7 MPa)**

| Composition de la charge (% propylène; % propane) | Différence de pression entre les faces amont et aval du film | Température du film | Perméabilité C3H6 | Perméabilité C₃H₆ / Perméabilité C₃H₈ (sélectivité en mélange) |
|---|---|---|---|---|
| mélange 90/10 | 0,8 MPa | 60°C | 17 barrer | 5 |
| mélange 70/30 | 0,8 MPa | 60°C | 18 barrer | 4,5 |

### Exemple 9 (selon l'invention)

Les membranes synthétisées selon les exemples 1 à 3 selon l'invention, ont également été testées pour séparer l'éthylène de l'éthane.

Un échantillon de chaque membrane a été testé dans une cellule de perméation circulaire d'un diamètre efficace de 5,5 cm placé dans une enceinte thermostatée.

La face amont de la membrane ainsi testée est balayée pendant 48h avec un flux gazeux de 10 Nl/h composé de d'éthylène et d'éthane, à une fraction molaire respective de 90% et 10%, alors que le compartiment en aval de la membrane, dans lequel est collecté le perméat, est balayé par un flux d'azote de 1 Nl/h à la pression atmosphérique.

La composition des différents fluides entrant et sortant des différents compartiments de la cellule de perméation est obtenue par chromatographie en phase gazeuse.

Les performances des membranes synthétisées selon les exemples 1,2 et 3 sont détaillées dans le tableau n°13.

**Tableau 13. Performances du polyimide 6FDA-BDAF (Pression charge = 0,9 MPa; mélange à séparer constitué de 90% molaire d'éthylène et 10% d'éthane)**

| Modèle de membrane | Différence de pression entre les faces amont et aval du film | Température du film | Perméabilité C₂H₆ (barrer) | Perméabilité C₂H₆ / Perméabilité C₂H₈ (sélectivité en mélange) |
|---|---|---|---|---|
| synthétisé selon le mode détaillé dans l'exemple n° 1 | 0,8 MPa | 60°C | 3 | 3,1 |
| synthétisé selon le mode détaillé dans l'exemple n°2 | 0,8 MPa | 60°C | 2,6 | 3,4 |
| synthétisé selon le mode détaillé dans l'exemple n°3 | 0,8 MPa | 60°C | 2,3 | 4,2 |

Il apparaît clairement que les films décrits selon les exemples n°1 à 3 offrent une sélectivité éthylène/éthane significative tout en conservant une perméabilité vis à vis de l'éthylène importante, supérieure à 1 barrer. Clairement, le post-traitement thermique de ce type de membrane conduit à une augmentation de la sélectivité de la membrane.

### Exemple 10 (selon l'invention)

Une membrane composite offrant une sélectivité oléfine/paraffine mettant en oeuvre une couche sélective selon le mode décrit dans la présente invention a été synthétisée en enduisant des fibres creuses en polyoxyde de phénylène (PPO) produite par la société Parker Filtration (Parker Hannifin SA, UCC France, Rue Albert Calmette, BP6, 41260 La Chaussée St Victor, France), selon le mode suivant :
Le polymère de type 6FDA-BDAF obtenu selon la méthode n°2 est mis en solution dans le DMAC à une concentration massique de 8% sous l'effet d'une bonne agitation mécanique à température ambiante. La solution limpide est ensuite filtrée sous une pression de 0,2 MPa sur un filtre de type Millipore ayant un seuil de coupure de 1 µm. On trempe la fibre en poly-2,6-diméthyl-1,4-phénylène oxyde dans la solution de polymère dilué, puis on l'extrait verticalement de la solution en prenant soin que l'excès de solution soit évacuée par gravité.

La fibre enduite est ensuite mise à sécher verticalement dans une étude sous atmosphère inerte selon le programme thermique suivant : 30 min à 100 °C, 2 heures à 160 °C.

Des échantillons de fibres enduites analysées par microscopie électronique à balayage ont montré que la couche de polyimide sélectif aux oléfines présentait une épaisseur comprise entre 0,1 et 0, 5 µm.

Un faisceau de fibres est ensuite serti dans une calandre avec de la résine époxy et est soumis à des tests de séparation de mélange propylène/propane à l'état gazeux dans les conditions décrites dans l'exemple n°1. Lors de tests de séparation de mélanges constitués de propylène et de propane à des ratios molaires respectifs de 90% et 10%, à une température de 50°C et des pressions en amont et en aval de la membrane respectivement de 0,9 et 0,1 MPa, la sélectivité en mélange des fibres composites est de 6,5.

## Revendications

1. Procédé de séparation par membrane permettant la séparation sélective d'une oléfine contenue dans un mélange d'autres hydrocarbures de nombre d'atomes de carbone voisin de celui de l'oléfine à séparer, la couche sélective de ladite membrane étant constituée d'un film dense polymère dont la structure chimique contient un groupement bis-phényl-9,9-fluorène.

2. Procédé de séparation par membrane selon la revendication 1 dans lequel la couche sélective de la membrane polymère contenant le groupement bis-phényl-9,9-fluorène est sélectionnée dans le groupe constitué par les polymères des familles suivantes: les polyimides, les polyamides, les polycarbonates, les polysulfones, les poly(amides imides), les poly(éther sulfones), les polyesters, ou par les copolymères ou mélanges de polymères de ces familles.

3. Procédé de séparation par membrane selon la revendication 1 dans lequel la couche sélective de la membrane polymère contenant le groupement bis-phényl-9,9-fluorène appartient à la famille des polyimides.

4. Procédé de séparation par membrane selon la revendication 1 dans lequel la couche sélective de la membrane polymère contenant le groupement bis-phényl-9,9-fluorène appartient à la famille des polyamides.

5. Procédé de séparation par membrane selon la revendication 1 dans lequel la couche sélective de la membrane polymère contenant le groupement bis-phényl-9,9-fluorène appartient à la famille des polycarbonates.

6. Procédé de séparation par membrane selon la revendication 1 dans lequel la couche sélective de la membrane polymère contenant le groupement bis-phényl-9,9-fluorène appartient à la famille des polysulfones.

7. Procédé de séparation par membrane selon la revendication 3, dans lequel le polymère constitutif de la couche sélective de la membrane est un polyimide ayant été synthétisé avec le dianhydride de l'acide 2,2- bis(3,4-dicarboxyphényl)hexafluoropropane.

8. Procédé de séparation par membrane selon la revendication 3, dans lequel le polymère constitutif de la couche sélective de la membrane est un polyimide ayant été synthétisé avec le dianhydride de l'acide 3,3',4,4'-biphényltétracarboxylique.

9. Procédé de séparation par membrane selon la revendication 3, dans lequel le polymère constitutif de la couche sélective de la membrane est un polyimide ayant été synthétisé avec le dianhydride de l'acide 3,3',4,4'-benzophénonetétracarboxylique.

10. Procédé de séparation par membrane selon la revendication 3, dans lequel le polymère constitutif de la couche sélective de la membrane est un polyimide ayant été synthétisé avec la diamine 1,3-diamino-2,4,6-triméthylbenzène.

11. Procédé de séparation par membrane selon la revendication 3 dans lequel le polymère constitutif de la couche sélective de la membrane est un polyimide issu de la polycondensation du dianhydride de l'acide 2,2- bis(3,4-dicarboxyphényl)hexafluoropropane et de la diamine 9,9-bis(4-aminophényl)fluorène.

12. Procédé selon la revendication 3, dans lequel le polymère constitutif de la couche sélective de la membrane est un polyimide issu de la polycondensation du dianhydride de l'acide 3,3',4,4'-biphényltétracarboxylique et de la diamine 9,9-bis(4-aminophényl)fluorène.

13. Procédé selon la revendication 3, dans lequel le polymère constitutif de la couche sélective de la membrane est un polyimide issu de la polycondensation du dianhydride de l'acide 3,3',4,4'-benzophénonetétracarboxylique et de la diamine 9,9-bis(4-aminophényl)fluorène.

14. Procédé selon la revendication 3, dans lequel le polymère constitutif de la couche sélective de la membrane est un polyimide issu de la polycondensation d'une part du dianhydride de l'acide 2,2- bis(3,4-dicarboxyphényl)hexafluoropropane, et d'autre part d'un mélange de la diamine 9,9-bis(4-aminophényl)fluorène et de la diamine 1,3-diamino-2,4,6-triméthylbenzène.

15. Procédé selon la revendication 3, dans lequel le polymère constitutif de la couche sélective de la membrane est un polyimide issu de la polycondensation d'une part du dianhydride de l'acide 3,3',4,4'-biphényltétracarboxylique, et d'autre part d'un mélange de la diamine 9,9-bis(4-aminophényl)fluorène et de la diamine 1,3-diamino-2,4,6-triméthylbenzène.

16. Procédé selon la revendication 3, dans lequel le polymère constitutif de la couche sélective de la membrane est un polyimide issu de la polycondensation d'une part du dianhydride d de l'acide 3,3',4,4'-benzophénonetétracarboxylique et d'autre part d'un mélange de la diamine 9,9-bis(4-aminophényl)fluorène et de la diamine 1,3-diamino-2,4,6-triméthylbenzène.

17. Procédé selon l'une quelconque des revendications 1 à 16 dans lequel l'oléfine à séparer du mélange d'hydrocarbure est de l'éthylène.

18. Procédé selon l'une quelconque des revendications 1 à 16 dans lequel l'oléfine à séparer du mélange d'hydrocarbures est du propylène.

19. Procédé selon l'une quelconque des revendications 1 à 18 dans lequel la pression partielle d'oléfine est supérieure à 0,3 MPa.

20. Procédé selon l'une quelconque des revendications 1 à 19 dans lequel le film constituant la couche sélective de la membrane polymère a subi un post-traitement thermique à une température supérieure à 250°C pendant au moins une heure, destiné à augmenter la sélectivité du film polymère.

21. Procédé selon l'une quelconque des revendications 1 à 20 dans laquelle la couche sélective est déposée à la surface d'un support de type fibre creuse à base de PPO (poly-2,6- diméthyl-1,4-phénylène oxyde).

22. Procédé de séparation par membrane selon l'une quelconque des revendications 1 à 21 dans lequel la température du procédé est comprise entre -60 °C et 100 °C, et la pression du mélange à séparer est comprise entre 0, 1 et 5MPa.

23. Procédé de séparation par membrane selon l'une quelconque des revendications 1 à 21 dans lequel la température du procédé est comprise entre 30°C et 80 °C, et la pression du mélange à séparer est comprise entre 1 et 3 MPa.

24. Procédé de séparation par membrane selon l'une quelconque des revendications 1 à 21 dans lequel la température du procédé est comprise entre 40°C et 70 °C, et la pression du mélange à séparer est comprise entre 1 et 2 MPa.

## Claims

1. Process for membrane separation that allows the selective separation of an olefin that is contained in a mixture of other hydrocarbons with a number of carbon atoms that is close to that of an olefin that is to be separated, whereby the selective layer of said membrane consists of a dense polymer film whose chemical structure contains a bis-phenyl-9,9-fluorene group.

2. Process for membrane separation according to claim 1, in which the selective layer of the polymer membrane that contains the bis-phenyl-9,9-fluorene group is selected from the group that consists of the polymers of the following families: the polyimides, the polyamides, the polycarbonates, the polysulfones, the poly(amide imides), the poly(ether sulfones), the polyesters, or the copolymers or polymer mixtures of these families.

3. Process for membrane separation according to claim 1, in which the selective layer of the polymer membrane that contains the bis-phenyl-9,9-fluorene group belongs to the family of polyimides.

4. Process for membrane separation according to claim 1, in which the selective layer of the polymer membrane that contains the bis-phenyl-9,9-fluorene group belongs to the family of polyamides.

5. Process for membrane separation according to claim 1, in which the selective layer of the polymer membrane that contains the bis-phenyl-9,9-fluorene group belongs to the family of polycarbonates.

6. Process for membrane separation according to claim 1, in which the selective layer of the polymer membrane that contains the bis-phenyl-9,9-fluorene group belongs to the family of polysulfones.

7. Process for membrane separation according to claim 3, in which the polymer that constitutes the selective layer of the membrane is a polyimide that has been synthesized with the 2,2-bis(3,4-dicarboxyphenyl)hexafluoropropanoic acid dianhydride.

8. Process for membrane separation according to claim 3, in which the polymer that constitutes the selective layer of the membrane is a polyimide that has been synthesized with the 3,3',4,4'-biphenyltetracarboxylic acid dianhydride.

9. Process for membrane separation according to claim 3, in which the polymer that constitutes the selective layer of the membrane is a polyimide that has been synthesized with the 3,3',4,4'-benzophenonetetracarboxylic acid dianhydride.

10. Process for membrane separation according to claim 3, in which the polymer that constitutes the selective layer of the membrane is a polyimide that has been synthesized with the 1,3-diamino-2,4,6-trimethylbenzene diamine.

11. Process for membrane separation according to claim 3, in which the polymer that constitutes the selective layer of the membrane is a polyimide that is obtained from the polycondensation of the 2,2-bis(3,4-dicarboxyphenyl)-hexafluoropropanoic acid dianhydride and the 9,9-bis(4-aminophenyl)fluorene diamine.

12. Process according to claim 3, in which the polymer that constitutes the selective layer of the membrane is a polyimide that is obtained from the polycondensation of the 3,3',4,4'-biphenyltetracarboxylic acid dianhydride and the 9,9-bis(4-aminophenyl)fluorene diamine.

13. Process according to claim 3, in which the polymer that constitutes the selective layer of the membrane is a polyimide that is obtained from the polycondensation of the 3,3',4,4'-benzophenone-tetracarboxylic acid dianhydride and the 9,9-bis(4-aminophenyl)fluorene diamine.

14. Process according to claim 3, in which the polymer that constitutes the selective layer of the membrane is a polyimide that is obtained from the polycondensation, on the one hand, of a 2,2-bis(3,4-dicarboxyphenyl)-hexafluoropropanoic acid dianhydride, and, on the other hand, of a mixture of the 9,9-bis(4-aminophenyl)fluorene diamine and the 1,3-diamino-2,4,6-trimethylbenzene diamine.

15. Process according to claim 3, in which the polymer that constitutes the selective layer of the membrane is a polyimide that is obtained from the polycondensation of, on the one hand, 3,3',4,4'-biphenyltetracarboxylic acid dianhydride, and, on the other hand, a mixture of 9,9-bis(4-aminophenyl)fluorene diamine and 1,3-diamino-2,4,6-trimetylbenzene diamine.

16. Process according to claim 3, in which the polymer that constitutes the selective layer of the membrane is a polyimide that is obtained from the polycondensation, on the one hand, of the 3,3',4,4'-benzophenonetetracarboxylic acid dianhydride d and, on the other hand, of a mixture of the 9,9-bis(4-aminophenyl)fluorene diamine and the 1,3-diamino-2,4,6-trimethylbenzene diamine.

17. Process according to any of claims 1 to 16, in which the olefin that is to be separated from the hydrocarbon mixture is ethylene.

18. Process according to any of claims 1 to 16, in which the olefin that is to be separated from the hydrocarbon mixture is propylene.

19. Process according to any of claims 1 to 18, in which the olefin partial pressure is greater than 0.3 MPa.

20. Process according to any of claims 1 to 19, in which the film that constitutes the selective layer of the polymer membrane has undergone a post heat treatment at a temperature of more than 250°C for at least one hour, designed to increase the selectivity of the polymer film.

21. Process according to any of claims 1 to 20, in which the selective layer is deposited on the surface of a hollow-fiber-type substrate that is based on PPO (poly-2,6-dimethyl-1,4-phenylene oxide).

22. Process for membrane separation according to any of claims 1 to 21, in which the temperature of the process is between -60°C and 100°C, and the pressure of the mixture that is to be separated is between 0.1 and 5 MPa.

23. Process for membrane separation according to any of claims 1 to 21, in which the temperature of the process is between 30°C and 80°C, and the pressure of the mixture that is to be separated is between 1 and 3 MPa.

24. Process for membrane separation according to any of claims 1 to 21, in which the temperature of the process is between 40°C and 70°C, and the pressure of the mixture that is to be separated is between 1 and 2 MPa.

## Patentansprüche

1. Membrantrennverfahren, das das selektive Trennen eines Olefins ermöglicht, das in einem Gemisch aus anderen Kohlenwasserstoffen mit einer Anzahl von Kohlenstoffatomen nahe derjenigen der zu trennenden Olefine enthalten ist, wobei die selektive Schicht der Membran aus einem dichten Polymerfilm besteht, dessen chemische Struktur eine Biphenyl-9,9-fluoren-Gruppe enthält.

2. Membrantrennverfahren nach Anspruch 1, wobei die selektive Schicht der Polymermembran, die die Biphenyl-9,9-fluoren-Gruppe enthält, ausgewählt ist aus der Gruppe bestehend aus den Polymeren der folgenden Familien: den Polyimiden, den Polyamiden, den Polycarbonaten, den Polysulfonen, den Poly(amidimiden), den Poly(ethersulfonen), den Polyestern oder aus den Copolymeren oder den Gemischen dieser Familien.

3. Membrantrennverfahren nach Anspruch 1, wobei die selektive Schicht der Polymermembran, die die Biphenyl-9,9-fluoren-Gruppe enthält, zur Familie der Polyimide gehört.

4. Membrantrennverfahren nach Anspruch 1, wobei die selektive Schicht der Polymermembran, die die Biphenyl-9,9-fluoren-Gruppe enthält, zur Familie der Polyamide gehört.

5. Membrantrennverfahren nach Anspruch 1, wobei die selektive Schicht der Polymermembran, die die Biphenyl-9,9-fluoren-Gruppe enthält, zur Familie der Polycarbonate gehört.

6. Membrantrennverfahren nach Anspruch 1, wobei die selektive Schicht der Polymermembran, die die Biphenyl-9,9-fluoren-Gruppe enthält, zur Familie der Polysulfone gehört.

7. Membrantrennverfahren nach Anspruch 3, wobei das Polymer, das die selektive Schicht der Membran bildet, ein Polyimid ist, das mit 2,2-Bis(3,4- dicarboxyphenyl)hexafluorpropansäuredianhydrid synthetisiert wurde.

8. Membrantrennverfahren nach Anspruch 3, wobei das Polymer, das die selektive Schicht der Membran bildet, ein Polyimid ist, das mit 3,3',4,4'-Biphenyltetracarbonsäuredianhydrid synthetisiert wurde.

9. Membrantrennverfahren nach Anspruch 3, wobei das Polymer, das die selektive Schicht der Membran bildet, ein Polyimid ist, das mit 3,3',4,4'-Benzophenontetracarbonsäuredianhydrid synthetisiert wurde.

10. Membrantrennverfahren nach Anspruch 3, wobei das Polymer, das die selektive Schicht der Membran bildet, ein Polyimid ist, das mit 1,3-Diamino-2,4,6-trimethylbenzoldiamin synthetisiert wurde.

11. Membrantrennverfahren nach Anspruch 3, wobei das Polymer, das die selektive Schicht der Membran bildet, ein Polyimid ist, das aus der Polykondensation von 2,2-Bis(3,4-dicarboxyphenyl)hexafluorpropansäuredianhydrid und 9,9-Bis(4-aminophenyl)fluorendiamin stammt.

12. Verfahren nach Anspruch 3, wobei das Polymer, das die selektive Schicht der Membran bildet, ein Polyimid ist, das aus der Polykondensation von 3,3',4,4'-Biphenyltetracarbonsäuredianhydrid und 9,9-Bis(4-aminophenyl)fluorendiamin stammt.

13. Verfahren nach Anspruch 3, wobei das Polymer, das die selektive Schicht der Membran bildet, ein Polyimid ist, das aus der Polykondensation von 3,3',4,4'-Benzophenontetracarbonsäuredianhydrid und 9,9-Bis(4-aminophenyl)fluorendiamin stammt.

14. Verfahren nach Anspruch 3, wobei das Polymer, das die selektive Schicht der Membran bildet, ein Polyimid ist, das aus der Polykondensation von einerseits 2,2-Bis(3,4-dicarboxyphenyl)hexafluorpropansäuredianhydrid und andererseits einem Gemisch aus 9,9-Bis(4-aminophenyl)fluorendiamin und 1,3-Diamino-2,4,6-trimethylbenzoldiamin stammt.

15. Verfahren nach Anspruch 3, wobei das Polymer, das die selektive Schicht der Membran bildet, ein Polyimid ist, das aus der Polykondensation von einerseits 3,3',4,4'-Biphenyltetracarbonsäuredianhydrid und andererseits einem Gemisch aus 9,9-Bis(4-aminophenyl)fluorendiamin und 1,3-Diamino-2,4,6-trimethylbenzoldiamin stammt.

16. Verfahren nach Anspruch 3, wobei das Polymer, das die selektive Schicht der Membran bildet, ein Polyimid ist, das aus der Polykondensation von einerseits 3,3',4,4'-Benzophenontetracarbonsäuredianhydrid und andererseits einem Gemisch aus 9,9-Bis(4-aminophenyl)fluorendiamin und 1,3-Diamino-2,4,6-trimethylbenzoldiamin stammt.

17. Verfahren nach irgendeinem der Ansprüche 1 bis 16, wobei das von dem Kohlenwasserstoffgemisch zu trennende Olefin Ethylen ist.

18. Verfahren nach irgendeinem der Ansprüche 1 bis 16, wobei das von dem Kohlenwasserstoffgemisch zu trennende Olefin Propylen ist.

19. Verfahren nach irgendeinem der Ansprüche 1 bis 18, wobei der Partialdruck des Olefins größer als 0,3 MPa ist.

20. Verfahren nach irgendeinem der Ansprüche 1 bis 19, wobei der Film, der die selektive Schicht der Polymermembran bildet, mindestens eine Stunde lang einer thermischen Nachbehandlung bei einer Temperatur von mehr als 250 °C unterzogen wurde, die dazu dient, die Selektivität des Polymerfilms zu erhöhen.

21. Verfahren nach irgendeinem der Ansprüche 1 bis 20, wobei die selektive Schicht auf der Oberfläche eines Trägers vom Hohlfasertyp auf der Basis von PPO (Poly-2,6-dimethyl-1,4-phenylenoxid) abgeschieden wird.

22. Membrantrennverfahren nach irgendeinem der Ansprüche 1 bis 21, wobei die Verfahrenstemperatur im Bereich zwischen -60 °C und 100 °C und der Druck des zu trennenden Gemischs im Bereich zwischen 0,1 und 5 MPa liegen.

23. Membrantrennverfahren nach irgendeinem der Ansprüche 1 bis 21, wobei die Verfahrenstemperatur im Bereich zwischen 30 °C und 80 °C und der Druck des zu trennenden Gemischs im Bereich zwischen 1 und 3 MPa liegen.

24. Membrantrennverfahren nach irgendeinem der Ansprüche 1 bis 21, wobei die Verfahrenstemperatur im Bereich zwischen 40 °C und 70 °C und der Druck des zu trennenden Gemischs im Bereich zwischen 1 und 2 MPa liegen.
